**Europäisches Patentamt**

**European Patent Office**

**Office européen des brevets**

(11) Publication number: **0 153 154**
**B1**

# EUROPEAN PATENT SPECIFICATION

(45) Date of publication of patent specification: **16.05.90**

(21) Application number: **85300979.3**

(22) Date of filing: **14.02.85**

(51) Int. Cl.⁵: **C 12 N 15/00,** C 12 P 19/34,
C 12 N 1/20 // C12R1:19

(54) Transfer vector.

(30) Priority: **14.02.84 US 580445**

(43) Date of publication of application:
**28.08.85 Bulletin 85/35**

(45) Publication of the grant of the patent:
**16.05.90 Bulletin 90/20**

(84) Designated Contracting States:
**AT BE CH DE FR GB IT LI LU NL SE**

(56) References cited:
**EP-A-0 067 540**
**EP-A-0 073 635**

**Cell 20, p. 269-81 (1980)**

(73) Proprietor: **LUBRIZOL GENETICS INC.**
**3375 Mitchell Lane**
**Boulder Colorado 80301-2244 (US)**

(72) Inventor: **Ahlquist, Paul**
**3106 Bluff Street**
**Madison Wisconsin 53705 (US)**

(74) Representative: **Fisher, Adrian John et al**
**CARPMAELS & RANSFORD 43 Bloomsbury**
**Square**
**London WC1A 2RA (GB)**

**The file contains technical information
submitted after the application was filed and
not included in this specification**

Courier Press, Leamington Spa, England.

**Description**

The nucleotide sequences of a DNA molecule carry an information code which can be transcribed into an intermediate messenger (mRNA) followed by translation into a protein. Such a protein may have a catalytic or a structural function in the organism. In some cases the nucleotide sequence is transcribed into an RNA molecule and this RNA molecule is the functional entity. Natural examples are the ribosomal RNA molecules (rRNA), transfer RNA molecules (tRNA) and small nuclear RNA molecules (snRNA). Some viruses (e.g., retroviruses) are reverse transcribed and the DNA product is then incorporated into the host genome from whence it can be transcribed once again into viral RNA. A useful artificial situation would be to isolate a DNA fragment of exactly the same sequence as a useful RNA molecule and then to insert such a fragment precisely at the transcription initiation site downstream from a strong promoter sequence which in turn has been inserted into a vector plasmid. In such a case large quantities of the desired RNA molecule could be produced by *in vitro* transcription. Prior vector systems have provided insertion sites downstream from a promoter, but the RNA transcribed therefrom contains extraneous sequence at the 5' end. The function of the resulting RNA may be modified in an undesired or uncontrolled manner since many RNA functions are effected by the nucleotide sequence at the 5' end. This prior art difficulty could be overcome by a transcription vector providing for precise initiation of transcription of the inserted DNA segment. In this manner it is possible to produce wild type viral RNA sequences, mutated viral RNA sequences, viral RNA sequences in which some non-essential nucleotide sequences have been replaced by useful foreign genes and wild type or mutated rRNA, tRNA or snRNA molecules. Mutated viral RNA sequences may be used to infect plant cells thereby preventing superinfection or viral RNA sequences partly replaced by foreign genes may be used as vehicles for the introduction of those genes into plant cells. Alternatively the metabolism of microbial populations may be disrupted by transformation of the population with the recombinant plasmid carrying mutated rRNA, tRNA or snRNA. A method of constructing a recombinant plasmid carrying a vector, a strong promoter and a restriction endonuclease site at the transcription initiation point is described. This restriction endonuclease site is used to insert the desired DNA fragment.

Summary of the invention

The techniques of recombinant DNA have made it possible to transcribe and translate a variety of open reading frames in a DNA sequence. The efficiency of such transcription and translation depends on many factors, e.g., the "strength" of the promoter region, the structure of DNA sequences upstream from the transcription initiation site, and, in some cases, the DNA structure on the 3'-side of the termination signal. In some cases it is useful to produce an RNA transcript with precise initiation and termination points, e.g., for the *in vitro* production of viral RNA molecules, small nuclear RNA molecules and rRNA molecules. This invention describes the production by use of advanced genetic engineering techniques of a recombinant plasmid containing a blunt-ended restriction endonuclease site at a point downstream from a strong lambda virus promoter (PR) where transcription from various DNA sequences can be precisely initiated. In addition an *Eco*RI restriction site occurs immediately after the transcription initiation site so the use of *Eco*RI immediately prior to transcription *in vitro* will also precisely locate the termination point (within 7 nucleotides) of any DNA fragment inserted into the initiation restriction endonuclease site.

Background of the invention

In the past several years a very large potential has developed in the field of genetic engineering for the transfer of foreign genes to plants. In order to achieve such a transfer, it is necessary to find suitable vectors. The most advanced work on transfer of genes to a plant genome has been achieved by the use of *Agrobacterium tumefaciens* and, to a lesser extent, *Agrobacterium rhizogenes* (Leemans, J., *et al.*, (1982) In Kahl, G. and J. S. Schell (1982) Molecular Biology of Plant Tumors Ch. 21:537—545, Academic Press, New York). A recent study (Murai, N., *et al.* (1983) Science *222*:476—482) showed the sequences coding for the bean seed protein phaseolin were inserted into the sunflower genome by the transferred DNA regions of tumor inducing plasmids. In one instance the phaseolin encoding sequences were controlled by the octopine synthase promoter and in another instance by the phaseolin promoter region. In both cases the phaseolin genes were correctly transcribed, processed and translated, thus demonstrating the expression of a plant gene after transfer to a taxonomically distinct botanical family.

In addition, there is the potential of genetically engineering the normal plasmids of *Rhizobium* strains prior to the infection and formation of nodules on plant roots. Much work has already been done on the structure and function of nitrogenase genes (Scott, K. F., Rolfe, B. G. and J. Shine (1983) DNA *2*:141—148; Scott, K. F., Rolfe, B. G. and J. Shine (1983) DNA *2*:149—156). The promoter regions of the nitrogenase genes of these organisms have been used to control the transcription and translation of a variety of foreign genes inserted into the symbiotic plasmids of *Rhizobium* in such a manner as to be under the control of these promoter regions (U.S. Patent Application Serial No. 506,676, filed June 22, 1983). Both types of vectors, i.e., the T-DNA of *Agrobacterium* spp. and the symbiotic plasmids of *Rhizobium* spp. involve the transfer of foreign genes as DNA.

Another possibility for the transfer of foreign genes to plant cells is by the use of RNA or DNA viruses. In the case of RNA viruses, it would be necessary to reverse transcribe them into cDNA molecules because of the technical difficulties of handling RNA molecules and the lack of restriction endonuclease active on RNA. In some cases where amplification by secondary infection cycles is possible, e.g., poliovirus (Racaniello, V. and D. Baltimore (1981) Science *214*:916—919) and potato spindle tuber virus (Cress, D., Kiefer, M. and R. A. Owens (1983) Nucleic Acids Res. *11*:6821—6835), the method appears feasible but in many examples, infectivity of viral cDNA molecules is low or nonexistent, and this approach does not produce results. To understand why these negative results may occur, it is useful to list the following probable requirements for infectivity of directly-inoculated viral cDNA.

(1) cDNA uptake into the cell; (2) cDNA transport into the nucleus; (3) transcription of (at least) full-length viral RNA's (vRNA); (4) avoidance of vRNA splicing; (5) processing of vRNA termini to infectious forms; (6) vRNA transport to the cytoplasm; (7) translation of (at least some) viral proteins; and (8) effective interaction of vRNA with viral and cellular proteins for a first round of replication.

Although effective transcription is often considered to be the most critical feature, viral cDNA infectivity probably involves a number of additional requirements. For example, linkage of viral cDNA to a strong plant promoter might be a useful strategy but would not give infectivity if the viral RNA contains a cryptic splice site which leads to its effective processing to a non-infectious form. Also, a number of steps, e.g. (4) to (6) above, involve cellular processes which are poorly understood. Consequently, the infectivity of directly-inoculated cDNA could be severely limited at a number of steps, e.g., vRNA transport to the cytoplasm. These defects of infectivity would be extremely difficult to identify and so the various defects would be very difficult to correct without significant advances in basic molecular and cellular biology.

Several features are needed to turn a fragment of nucleic acid into a plant vector: (1) The nucleic acid should be capable of being cloned so that useful quantities can be recovered; (2) The nucleic acid should be able to replicate within a plant cell and preferably, should be recognizable by selective methods; (3) It should not be pathogenic; (4) The nucleic acid must be capable of incorporating other nucleic acid sequences into its structure and of expressing such incorporated nucleic acid; and (5) If heritable changes of the transformed plant are wanted, then the vector or a derivative of the vector should be stably maintained from one generation to the next. Further, in the case of T-DNA, at some stage the transformed plant cell will have to be purified by cloning and regenerated into a plant. When these features are considered in relation to RNA plant viruses, a number of difficulties become apparent: (1) The techniques of genetically manipulating and recombining RNA are much more difficult than the techniques of genetically manipulating the recombining DNA; (2) Since most virus particles are packaged into protein capsids, there may be difficulties in accommodating more than limited quantities of "foreign" nucleic acid; (3) RNA viruses need to be replicated within the plant cell so there will be difficulties in recovery of genetically engineered RNA; (4) The strain of RNA virus used to infect the plant cells should cause minimal pathogenic effects.

Detailed description of the invention

The key feature of the present invention is the construction of a recombinant expression vector with a strong promoter element and a blunt-ended restriction endonuclease site positioned downstream from the promoter so that transcription initiation always occurs at a precise nucleotide. A critical feature of the present invention is the discovery that, following the conversion of viral RNA molecules to cDNA by the use of reverse transcriptase, these cDNA molecules can be transcribed *in vitro* into RNA. Furthermore, such viral RNA molecules transcribed *in vitro* from cDNA molecules are infectious, provided that transcription, initiation and transcription termination are essentially at the correct nucleotides.

To be useful in the transmission of foreign genes into plants, DNA fragments are incorporated into suitable positions on the cDNA molecule. For example, using brome mosaic virus which has a tripartite genome (i.e., RNA1, RNA2 and RNA3), it may be possible to delete the nucleic acid sequence coding for the coat protein in RNA3 and replace it with the sequence coding for a foreign gene. Such a deletion and replacement could most easily be done by use of the cDNA molecule.

This strategy has several attractions: (a) Gene 3a occurs as one of the two open reading frames of RNA3 and it is necessary for this gene to be present for viral RNA replication within plant cells. The requirement for gene 3a function would sever to maintain the engineered component in the virus; (b) The foreign protein would presumably be strongly expressed by the same mechanisms which lead to strong coat protein production in normal infection; (c) The size of the inserted sequences would not be limited by encapsidation constraints; (d) The lack of viral coat protein should limit the escape of engineered virus from deliberately inoculated plants. If both the 3a and coat genes (encoded by RNA3) were required for infection, they could be separated onto two different components, one or both of which could also carry foreign genetic information. Up to 2kb of foreign sequence should be insertable in a single such component without violating packaging constraints.

Many other viruses are useful in the *in vitro* production of infectious RNA molecules by transcription from cloned cDNA, including various other plant viruses [e.g., tobacco mosaic virus (TMV)], poliovirus, influenza virus, foot and mouth disease virus, and others.

There is yet another benefit to be obtained from the *in vitro* transcription of viral mRNA molecules from cDNA. It is possible to produce mutations in the viral cDNA molecules that convert a virulent strain to a symptomless strain. Such symptomless strains are used in *in vitro* synthesis of viral mRNA which is genetically engineered to contain foreign genes. Infection of plants by such symptomless strains of virus can also prevent later superinfection of these plants by virulent strains of virus.

Another use of a recombinant vector carrying a strong promoter and a blunt-ended restriction site positioned precisely at the start of transcription is the use of mutated rRNA molecules to combat bacterial infections. rRNA molecules are transcribed from rDNA. Thus, it is possible to mutate the 3'-end of the bacterial 16S rRNA molecule in such a way that the rRNA takes its place in the ribosomes but is incapable of mediating translation of mRNA molecules. Multiple transformation of bacteria by a recombinant vector containing such a mutated rRNA severely retards or eliminates bacterial infections. Similarly, mutated tRNA's which are transcribed from tDNA's could be used for the multiple transformation of bacterial populations. Such mutated tRNA's can cause a severe disruption of the bacterial capability to translate mRNA's into needed proteins.

Yet another use of the transfer vector described herein is the enhancement of translation of a mRNA transcribed from a eukaryotic gene in a transformed bacterium. It is well known in the art that tRNA populations of bacteria occur in different proportions compared to the tRNA populations of eukaryotes and that codon usage is different in the mRNA's of prokaryotes and eukaryotes. Translation of a mRNA transcribed from a eukaryotic gene in a transformed bacterium can be optimized by further transforming a variety of tDNA's under the precise control of a promoter into the transformed bacterium.

In order to clone these various DNA molecules from which precisely initiated and terminated transcripts are required, it is necessary to construct a recombinant plasmid where the DNA to be transcribed is inserted at a precise distance downstream from the regulatory signals of a strong bacterial or viral promoter. Bacteriophage promoters frequently provide strong initiation signals because an infecting virus must redirect the cellular metabolism of a host to its own purposes. It has been shown that lambda $P_R$ promoter initiates transcription many times more frequently than bacterial promoters such as *gal* or *lac* (Queen, C. and M. Rosenberg (1981) Cell *25*:241—249).

In previous work to increase the production of proteins (Queen, C. (1983) J. Mol. Appl. Genet. *2*:1—10) an expression vector was created by placing the $P_R$ promoter and *cro* gene ribosome binding site on a plasmid, along with a repressor gene for $P_R$ in such a way that the *cro* ribosome binding site could be easily linked to foreign genes. The effectiveness of this vector was shown by producing two proteins, β-galactosidase and SV-40 small t-antigen, at the level of 5—10% of total *E. coli* protein. The level of t-antigen achieved ($4 \times 10^5$ molecules/cell) was about 10 times higher than was obtained with the *lac* system (Thummel, S., Burgess, T. L. and R. Tijian (1981) J. Virol. *37*:683—697) or a chemically synthesized ribosome binding site (Jay, G., Khoury, G., Seth, A. and Jay, E. (1981) Proc. Nat. Acad. Sci. U.S.A. *78*:5543—5548) and is 20 times higher than the level of interferon obtained using a triple *trp* promoter (Goeddel, D. V. Shepard, H. M., Yelverton, E., Leung, D. and R. Crea (1980) Nucleic Acids Res. *8*:4057—4073).

This expression vector which carried the $P_R$ phage lambda promoter (Queen C. (1983) J. Mol. Appl. Genet. *2*:1—10) was the starting point for creating an entirely novel vector, namely, a vector carrying a strong promoter with a restriction site downstream from the regulatory sequences of the strong promoter precisely at the transcription initiation site. Since the restriction site can be used to insert foreign DNA, this means that transcription initiation of any foreign DNA can be precisely positioned.

In the construction of these transcription initiation vectors, one is not confined to the use of the $P_R$ lambda phage promoter. Any suitable promoter may be used; such a promoter may be strong or weak and subject to regulation by activators and/or repressors. A representative list of such promoters would include (but not be restricted to) the following list: (a) Bacterial promoters such as *gal*P1 (Musso, R. *et al.* (1977) Proc. Nat. Acad. Sci. U.S.A. *74*:106—110) or trp (Bennet, G. N. *et al.* (1978) J. Mol. Biol. *121*:113—137); (b) Other phage promoters such as T7 A1 (Siebenlist, U. (1979) Nucleic Acids Res. *6*:1895—1907), or those of phage SP6 (Green, M. *et al.* (1983) Cell *32*:681—694); (c) Plasmid and transposon promoters such as pBR322bla (Sutcliffe, J. G. (1979) Proc. Nat. Acad. Sci. U.S.A. *75*:3737—3741) and Tn5neo (Rothstein, S. J. *et al.* (1981) Cell *23*:191—199) and promoters created by fusion or mutation such as lacP115 (Johnson, R. C. *et al.* (1981) Nucleic Acids Res. *9*:1873—1883) and IS2 I-II (Hinton, D. M. and R. E. Musso (1982) Nucleic Acids Res. *10*:5015—5031). In addition, completely synthetic promoters or promoters recognized by RNA polymerases from biological sources other than bacteria may be used.

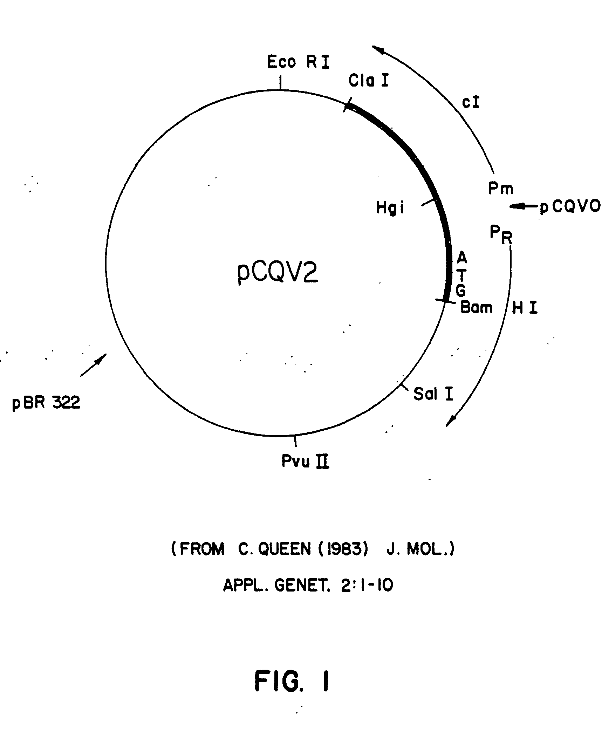

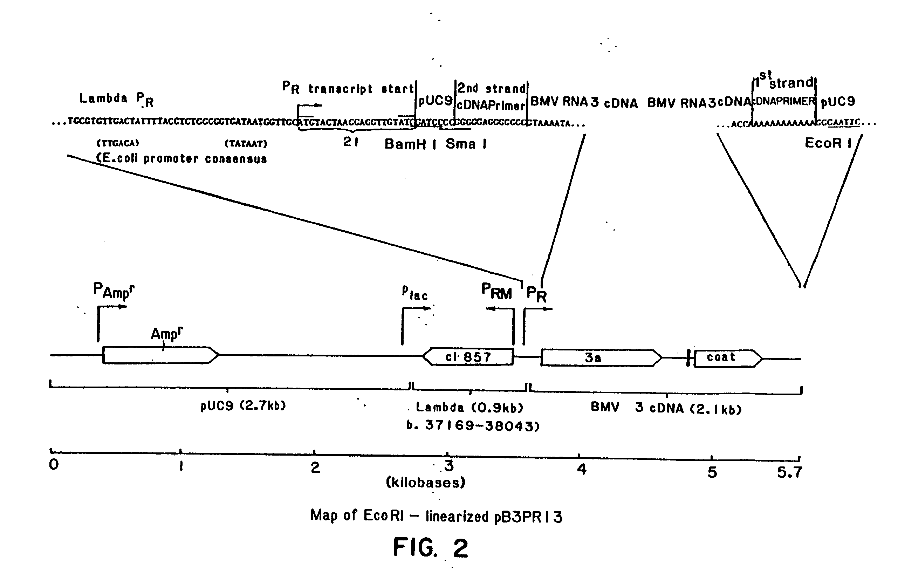

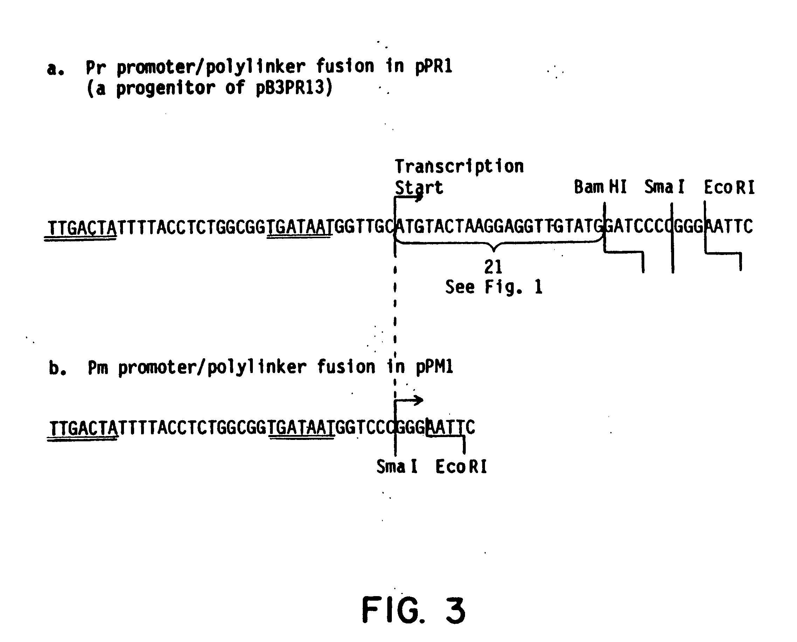

The vector used in the present invention for the insertion, propagation and manipulation of the *Bam*HI—*Cla*I DNA fragment from the expression vector pCQV2 (Fig. 1) was M13mp9 (Messing, J. and Vieira, J. (1982) Gene *19*:269—276). The M13mp9 in this example was chosen for ease and convenience. The use of this strain is not to be interpreted as excluding the use of other filamentous single stranded phages (e.g., f1 or fd). Other filamentous, single stranded phages could equally well be utilized in these experiments (The Single Stranded DNA phages (1978) Denhardt, D. T., Dressler, D. and D. S. Ray, eds Cold Spring Harbor Laboratory, New York). M13mp9 was derived from another M13 engineered phage, i.e., M13mp7. The cloning of DNA into the replicative form (RF) of M13 has been facilitated by a series of improvements which produced the M13mp7 cloning vehicle (Gronenborn, B. and J. Messing (1978) Nature, Lond. *272*:375—377; Messing, J. (1979) Recombinant DNA Technical Bulletin, NIH Publication No. 79—99, ·2, No. 2 43—48; Messing, J., Crea, R. and P. H. Seeburg (1981) Nucleic Acids Res. *9*:309—321). A fragment of the *E. coli lac* operon (the promoter and N-terminus of the β-galactosidase gene) was inserted into the M13 genome. This *lac* promoter-operator insert codes for the first 145 amino acid residues of the β-galactosidase gene. During the infection of certain cell lines (e.g., *E. coli* K12 JM101 and JM103) this information will complement certain deletion mutants of the β-galactosidase gene and restore β-

galactosidase activity (α-complemention). These cells show a *lac*[+] phenotype and can be identified by a blue colored plaque on a medium containing IPTG and X-*gal* (Malamy, M. H., Fiandt, M. and Szybalski, W. (1972) Mol. Gen. Genet. *119*:207ff). In addition, a small DNA fragment synghesized *in vitro* and containing an array of restriction cleavage sites [a multiple cloning site (MCS)] was inserted into the structural region of the β-galactosidase gene fragment. In spite of these insertions the M13mp7 DNA is still infective and the modified *lac* DNA is able to encode the synthesis of a functional β-galactosidase α-peptide (Langley, K. E., Villarejo, M. R., Fowler, A. V. Zamenhof, P. J. and I. Zabin (1975) Proc. Nat. Acad. Sci. U.S.A. *72*:1254—1257). This synthesized DNA fragment in M13mp7 contains two sites each for the *Eco*RI, *Bam*HI, *Sal*I, *Acc*I and *Hinc*II restriction enzymes arranged symmetrically with respect to a centrally located *Pst*I site. By chance, either strand of a cloned restriction fragment can become part of the viral (+) strand. This depends on the fragment orientation relative to the M13 genome after ligation. The insertion of a DNA fragment into one of these restriction sites is readily monitored because the insertion results in a non-functional α-peptide and the loss of β-galactosidase activity. Under appropriate conditions, e.g., when strains *E. coli* K12 JM101 or 103 are infected by M13mp7, the functional α-peptide results in blue plaques; a non-functional α-peptide results in colorless plaques (Messing, J. and B. Bronenborn (1978) In Denhardt, D. T., Dressler, D. and D. S. Ray (eds.) The Single Stranded DNA Phages. Cold Spring Harbor Laboratory, Cold Spring Harbor, New York, pp. 449—453). M13mp7 has found wide application in the dideoxy nucleotide sequencing procedure (Sanger, F., Nicklen, S. and A. R. Coulsen (1977) Proc. Nat. Acad. Sci. U.S.A. *74*:5463—5467).

A disadvantage of M13mp7 was that the multiple cloning site (MCS) contained two of each kind of restriction endonuclease site thus allowing DNA restriction fragments to be inserted in both orientations. Therefore, two new ssDNA bacteriophage vectors, M13mp8 and M13mp9, were constructed (Messing, J. and Vieira, J.(1982) Gene *19*:268—276). The nucleotide sequence of M13mp7, containing the multiple restriction sites, was modified to have only one copy of each restriction site and, in addition, single *Hind*III, *Sma*I and *Xma*I sites. Thus, DNA fragments whose ends correspond to two of these restriction sites can be "forced cloned" by ligation to one of these new M13 cloning vehicles that has also been "cut" with the same pair of restriction enzymes. M13mp8 and M13mp9 have the modified multiple restriction site region arranged in opposite orientations relative to the M13 genome and a given restriction fragment can be directly oriented by forced cloning. The use of both vectors M13mp8 and M13mp9 guarantees that each strand of the cloned fragment will become the (+) strand in one or the other of the clones and thus be extruded as ssDNA in phage particles.

Initially, fusion of the lambda phage $P_R$ promoter to the pUC9 polylinker resulted in 21 nucleotides of lambda sequence and some additional pUC9 nucleotides appearing at the 5'-end of the synthetic brome mosaic virus cDNA3 transcript (Figs. 2 and 3). In order to allow the production of synthetic RNA transcripts (including viral RNA transcripts) with correct 5'-termini, a "universal" transcription vector, now designated pPM1, was created. This transcription vector (pPM1) retained most elements of the $P_R$ promoter but the transcription initiation site coincided with the cut site of a unique blunt-ended restriction site (the *Sma*I site in Fig. 3b). The construction of such a transcription vector became possible when examination of a large number of promoter sequences revealed that it might be possible to generate a blunt-ended *Sma*I site at the position of transcription initiation by specific site directed mutagenesis without altering the promoter strength. Transcription of DNA fragments blunt-end ligated into this *Sma*I site was later demonstrated to begin at the first nucleotide of the inserted fragment. In particular, viral cDNA's were primed by terminally complementary oligonucleotides, blunt-end ligated into this site and transcribed to give the correct 5'-ends. Further, the initial form of this vector (Fig. 3b) also provided a unique *Eco*RI site immediately downstream of the *Sma*I site where transcription could be runoff-terminated to give product RNA's with no more than seven additional nucleotides at the 3' end.

To construct the pPM1 vector, the lambda cl-$P_{RM}$-$P_R$ cartridge from pCQV2 (Fig. 2) was subcloned into M13mp9. The resulting ssDNA was used as a template for DNA synthesis from a mismatched oligonucleotide designed to fuse the initiation site to the left half of the *Sma*I recognition sequence. The mismatched oligonucleotide was synthesized by an "in-house" facility. The DNA copied from the mismatch primer was made double stranded, isolated and ligated into M13mp9. After verifying the sequence of the modified $P_R$ promoter (i.e., PM promoter), the new cl-$P_{RM}$-$P_M$ cartridge was subcloned into pUC9 to give the final desired vector pPM1. Several non-trivial technical difficulties were encountered in the use of the mismatch primer. It was found at first that the primer was completely to a nucleotide sequence which was 96 residues upstream from the $P_R$ promoter and that successful priming was not achieved 3 nucleotides upstream from the $P_R$ promoter initiation site as was expected.

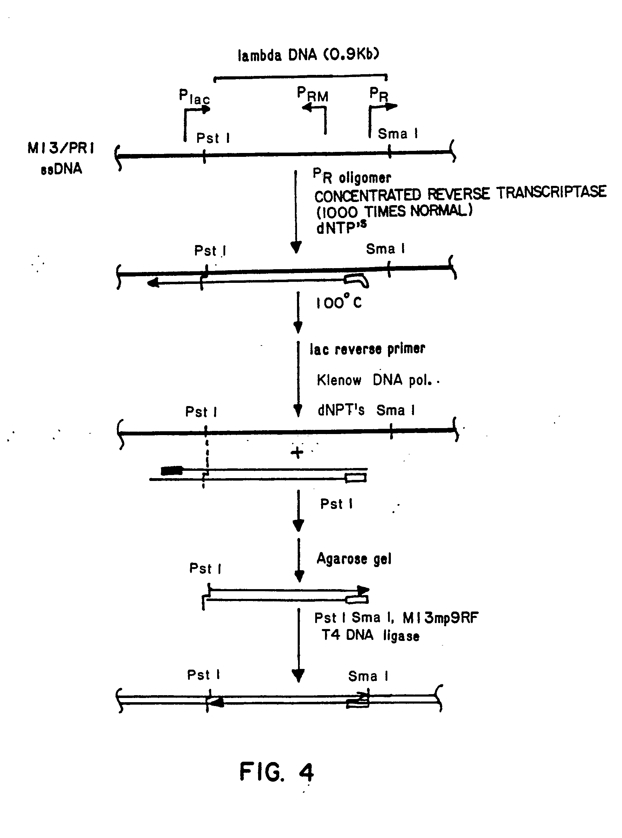

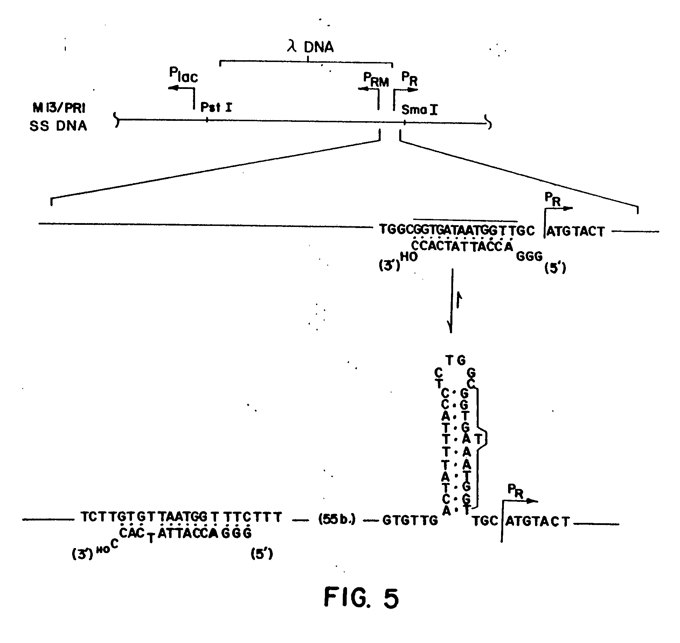

The solution to this unexpected result was finally reached after an exhaustive series of experiments which eliminated other possibilities and showed that the initial cloning protocol failed at the stage of oligomer binding. A comprehensive consideration of possible mechanisms of failure to bind oligomer at the desired site and a computer analysis of possible complementary pairing situations culminated in the conclusion that secondary structure in the template was the most likely problem (Fig. 5). This discovery then led to the development of a novel strategy to drive binding to the desired site despite competition from intra-molecular interactions. This novel strategy included use of reverse transcriptase in place of DNA polymerase I Klenow fragment (Fig. 4) and complete denaturation followed by slow renaturation of the template in the presence of a high effective concentration of the mismatched primer. Finally, direct testing of this newly developed strategy using a wide range of primer concentrations to determine the required levels of primer resulted in effective initiation of DNA synthesis at the desired position, namely, 3 nucleotides upstream from the $P_R$ promoter initiation site.

The recognition sequence of the *Sma*I restriction endonuclease is:

*Sma*I

| Promoter | 5'-CCC GGG-3' |
| --- | --- |

transcription start nucleotide

| "upstream" | 3'-GGG CCC-5' cleavage site | "downstream" | Reading strand |
| --- | --- | --- | --- |

The cleavage site occurs in the center between C and G. The reading strand is 3'-G-G-G-C-C-C-5'. The cleavage site of the *Sma*I restriction endonuclease is therefore on the 3'-side of the transcription initiation nucleotide with respect to the reading strand. Again, with respect to the reading strand, "upstream" is defined as 5'- - ->3' and "downstream" is defined as 3'- - ->5'.

In general, the critical feature in the construction of transcription initiation vectors is the distance between the promoter sequence and the blunt ended cleavage site of a restriction endonuclease. The cleavage site of the restriction endonuclease must be on the 3'-side of the nucleotide which is used for transcription initiation on the reading strand such that the cleavage site separates transcribed from untranscribed nucleotides. The term "transcription initiation nucleotide", as used herein, means the first transcribed nucleotide, i.e., the nucleotide corresponding to (or complementary to, depending on the DNA strand) the 5'-terminal ribonucleotide of the RNA transcript.

The next step is to clone extract cDNA copies of the various RNA molecules, i.e., the three genomic RNA's of brome mosaic virus or other viruses (see *supra*), the 16S RNA of various bacteria and the snRNA's from eukaryotic cells. These steps can be accomplished by synthesizing oligonucleotides complementary to each end of the RNA for use as first and second strand cDNA primers. Once the cDNA's have been synthesized and cloned into the unique *Sma*I site of the novel pPM1 vector plasmid, it is then possible to synthesize RNA which differ from their authentic natural RNA counterparts only by the addition of a maximum of seven nucleotides to the 3'-end.

Example 1
Construction and screening of phage recombinants M13/PR1—9

Plasmid pCQV2 (Queen (1983) J. Mol. Appl. Genet. *2*:1—10) was cleaved with restriction endonucleases *Cla*I and *Bam*HI and the fragment containing the phage lambda cl$^{ts}$-P$_{RM}$-P$_R$-ATG sequences was isolated from a low-melting point agarose gel (Sanger et al. (1980) J. Mol. Biol. 143:161—178) and ligated into calf intestinal phosphatased *Acc*I+*Bam*HI cut M13mp9 RF DNA using T4 DNA ligase. This DNA was used to transform competent *E. coli* JM101 cells and plated. Nine white plaques were selected and screened by the ddATP sequencing technique (Sanger *et al.* (1982) JMB *162*:729—773). The resulting autoradiograph confirmed that eight of the nine phage clones contained the expected phage lambda DNA insert, the remaining clone being a deletion mutant of M13mp9. One of the M13/λ recombinants, designated M13/PR1, was selected for further work.

Example 2
Optimum primer concentrations for mismatch priming on M13mp9/pPR1 ssDNA

The 15-mer mismatched oligonucleotide 5'-OH-d(GGGACCATTATCACC)-3'-OH which will be referred to as either the $P_R$-oligonucleotide or the $P_R$-primer, was synthesized by the phosphite triester method. This $P_R$-primer was designed in such a manner as to allow a precise alteration of the phage lambda $P_R$ promoter. A number of ways were used to test the priming of this $P_R$-primer on $P_R$ DNA. Beause of the presence of the complementary stem and loop in the nucleotide sequence of interest (see *supra*), the following experiments were designed to test whether complete denaturation of the M13mp9/pPR1 DNA template plus vast excesses of the $P_R$-primer could achieve priming at the lambda $P_R$ promoter and whether such primers annealed at this site could be extended by reverse transcriptase without extension of $P_R$-primers bound at the upstream cl$^{ts}$ site. It will be noted (Fig. 5) that when the $P_R$-primer is bound at the upstream cl$^{ts}$ site that it cannot then pair at the extreme 3'-terminus. Such a lack of pairing at the extreme 3'-terminus should prevent extension by reverse transcriptase which lacks a 3'-exonuclease activity. Therefore, M13/PR1 ssDNA was mixed with $P_R$ oligomer at 1, 10, 100, and 1000-fold molar excesses, annealed by the boiling/slow cooling method (Anderson *et al.* (1980) Nucle. Acids. Res. *8*:1731ff) and used as a template/primer complex in ddTTP sequencing reactions using reverse transcriptase (Ahlquist *et al.* (1981) Cell *23*:183—189). Autoradiography of the final gel showed that under these conditions DNA synthesis was primed exclusively from the desired site within the $P_R$ promoter sequence. Efficiency of DNA synthesis increased steadily with $P_R$ primer concentration, being at the highest primer:template ratio (1000:1) as efficient as synthesis from a 5-fold excess of the normal M13/lac sequencing primer (Duckworth *et al.*, Nucl. Acads. Res. *9*:1961—1706).

Example 3

Use of the PR oligonucleotide to synthesize and clone an altered promoter sequence

A 1000-fold molar excess of $P_R$ oligomer was mixed with approximately 2 µg M13/PR1 ssDNA and annealed by the boiling/slow cooling method (Anderson *et al.* (1980) Nucl. Acids Res. *8*:1731ff). The resulting template/primer complexes were extended by reverse transcriptase in the presence of all four dNTPs. The product DNA was isolated by ethanol precipitation and used in a second DNA synthesis reaction primed with an M13 lac "reverse" primer as described by Hong (Bioscience Reports *1*:243—252 (1981)). The resulting DNA products were cleaved with *Pst*I, electrophoresed on a low-melting point agarose gel, and the 800—1000 bp fraction, as assayed by the mobility of defined marker DNA fragments, excised and eluted (anger *et al.* (1980), J. Mol. Biol. *143*:161—178). This DNA was then mixed with *Sma*I+*Pst*I cut M13mp9 RF DNA, treated with T4 DNA ligase, and used to transform competent *E. coli* JM101 cells. After plating, 30 white plaques were selected and screened by the ddATP sequencing method (Sanger *et al.* (1982) JMB *162*:729—773). The pattern obtained for 16 of these recombinants was consistent with the desired construct in which the sequence of the lambda $P_R$ promoter element was linked directly to the vector *Sma*I site.

Since the purpose of the PM clone constructions was to obtain a particular altered promoter sequence (i.e., a *Sma*I restriction endonuclease site), two of these recombinants, designated M13 PM86 and M13 PM87, were sequenced by the 2′, 3′-dideoxynucleoside triphosphate method (Sanger, F. *et al.*, (1977) Proc. Nat. Acad. Sci. U.S.A. *74*:5463—5467). The nucleotide sequence in the vicinity of the altered $P_R$ promoter in both strains was exactly as desired, namely:

*Sma*I

...TGCGTGTTGACTATTTTACCTCTGGCGGTGATAATGGTCCCGGGAATTCACTGGCC...

$P_R$                        *Eco*RI

lambda sequence                  M13mp9 sequence

The nucleotide sequence changes that have been made at the *Sma*I site can be seen by a comparison of the above sequence with that of PB3PR13 (Fig. 2). The seqeunce of M13 PM86 was read for a further 210 bases upstream of the sequence shown above. It was then computer matched to the sequence of pCQV2 (Queen, C. (1983) J. Mol. Appl. Genet. *2*:1—10) without any discrepancies. Close visual examination of the autoradiograph revealed no sequence differences between M13 PM86 and M13 PM87.

Example 4

Subcloning M13/PM86,87 inserts into pUC9

For each of the two clones M13 PM86 and M13 PM87, RF DNA was digested with the restriction endonucleases *Pst*I and *Eco*RI and the *Eco*RI-*Pst*I fragment containing the desired modified $P_R$ promoter was recovered following electrophoresis in low melting point agarose. The appropgiate DNA band (as judged by size) was excised, the DNA extracted as previously described (*supra*), and ligated to *Eco*RI+*Pst*I cut pUC9 (Vieira and Messing (1982) Gene *19*:259—268). This DNA was used to transform competent *E. coli* MJ101 cells which were plated on media containing ampicillin, X-gal and IPTG. *Eco*RI and *Eco*RI+*Pst*I digests of minilysate DNA samples (Ish-Horowicz and Burke (1981) Nucl. Acids. Res. *9*:2989ff) from six white colonies from each ligation mix were examined by agraose gel electrophoresis. In each case the digests confirmed the expected size of the insert fragment and that only one such fragment had been inserted. One of these clones, an M13/PM86 derivative designated pPM1, was selected for further work. Transcripts of pPM1 were obtained by standard methods. Runoff reverse transcription of these transcripts showed that initiation on the $P_R$ promoter occurred precisely at the *Sma*I site as planned (illustrated below).

transcript start

TGATAATGGTCCCGGGAATTCACTGGCC

*Sma*I

Example 5

Insertion of a complete cDNA copy of brome mosaic virus RNA3 (BMV RNA3) into the *Sma*I restriction endonuclease cleavage site of pPM1

The nucleotide sequence of BMV RNS3 is known (Ahlquist, P. *et al.* (1981) J. Mol. Biol. *153*:23—28). Starting from this information, synthetic oligodeoxynucleotide primers for the first and second strands of BMV RNA3 cDNA were designed, synthesized and used to prime production of ds cDNA to BMV RNA3 (Fields and Winter (1982) Cell *28*:303—313). Full length ds cDNA was isolated from an agarose gel (*supra*)

EP 0 153 154 B1

and inserted into the *Sma*I site of pPM1. Clones in which the cDNA was oriented to give (+) strand transcripts (i.e., transcripts corresponding in polarity to encapsidated viral RNA) were selected after screening by restriction digestion. Plasmid DNA from such selected clones was cut with *Eco*RI and transcribed *in vitro* by standard methods. Results obtained using runoff reverse transcription of the transcripts showed that transcription initiation occurred on the first nucleotide downstream from the *Sma*I cleavage site of the pPM1 transcription vector.

**Claims**

1. A recombinant DNA vector comprising
(a) a replication origin and selectable marker and
(b) a nucleotide sequence containing a promoter, a transcription initiation site of which has been modified to introduce a non-naturally occurring blunt-ended restriction endonuclease recognition sequence positioned such that a cleavage site of said restriction endonuclease recognition sequence coincides with the transcription initiation site of said promoter.

2. A recombinant DNA vector according to Claim 1 wherein said restriction endonuclease recognition sequence is a *Sma*I restriction endonuclease recognition sequence.

3. A recombinant DNA vector according to any of Claims 1 to 3 wherein said promoter is a bacteriophage promoter.

4. A recombinant DNA vector according to Claim 4 wherein said promoter is the lambda $P_R$ promoter.

5. A recombinant DNA vector according to any of Claims 1 to 3 wherein said promoter is a bacterial promoter.

6. A recombinant DNA vector according to any preceding Claim wherein said replication origin and said selectable marker are those of pUC9 or M13mp9.

7. A recombinant DNA vector according to any preceding Claim wherein said vector further comprises a unique second restriction endonuclease recognition sequence positioned immediately 3' to said restriction endonuclease recognition sequence, the cleavage site of which coincides with said transcription initiation site.

8. A recombinant DNA vector according to Claim 7 wherein said unique second restriction endonuclease recognition sequence is an *Eco*RI restriction endonuclease recognition sequence.

9. The recombinant DNA vector pPM1 as defined in Example 4.

10. A bacterial strain containing and replicating therein a recombinant DNA vector according to any preceding Claim.

11. A bacterial strain according to Claim 11, wherein the strain which is transformed is *Escherichia coli* K12 JM101 or *Escherichia coli* K12 JM103.

12. A process for constructing a recombinant DNA vector comprising
(a) a replication origin and selectable marker and
(b) a nucleotide sequence containing a promoter,
wherein said process includes the step of modifying the transcription initiation site of said promoter to introduce a blunt-ended restriction endonuclease recognition sequence such that a cleavage site of said restriction endonuclease recognition sequence coincides with the transcription initiation site of said promoter.

13. A process according to Claim 13 comprising the steps of:
(a) isolating a DNA fragment containing a promoter, the transcription initiation site of which is known,
(b) inserting said DNA fragment containing a promoter into the replicative form of a filamentous single-stranded phage strain to produce a recombinant filamentous single-stranded phage,
(c) transforming a bacterial strain with said recombinant phage,
(d) isolating ssDNA of said recombinant phage excreted from said bacterial strain,
(e) synthesizing a mismatched oligonucleotide primer partially complementary to said promoter in the vicinity of said transcription initiation site and containing the complement of all or part of a restriction endonuclease recognition sequence, said complement of said restriction endonuclease recognition sequence or portion thereof being positioned within said oligonucleotide primer such that the cleavage site of said complement of said restriction endonuclease sequence coincides with said transcription initiation site,
(f) replicating said ssDNA using said oligonucleotide primer to produce a modified complementary DNA strand containing a modified promoter, said modified promoter being the promoter comprising the sequence complementary to said oligonucleotide primer,
(g) replicating said modified complementary strand to produce a double stranded DNA fragment containing said modified promoter,
(h) introducing said DNA fragment containing said modified promoter into a DNA vector plasmid which contains a replication origin and a selectable marker thereby constructing a universal transcription vector.

14. A process according to Claim 14 wherein said DNA fragment containing a promoter is a cl-$P_R$M-$P_R$ fragment from phage lambda.

15. A process according to Claim 14 wherein said filamentous single-stranded phage strain is M13mp9.

8

16. A process according to any of Claims 13 to 15 wherein said bacterial strain is *Escherichia coli* K12 JM101 or *Escherichia coli* K12 JM102.

17. A process according to Claim 14 wherein said oligonucleotide primer is 5'-OH-d(GGGACCATTAT-CACC)-3'-OH.

18. A process according to Claim 14 wherein said DNA vector plasmid is pUC9.

19. A method of making a recombinant DNA vector, comprising (a) providing a vector according to any of Claims 1 to 10 or a vector constructed according to the process of any of Claims 13 to 18, (b) cleaving said vector with said restriction endonuclease; and (c) inserting a fragment of DNA at the cleavage site of said restriction endonuclease recognition site, such that the first nucleotide that will be transcribed is the first nucleotide of the 5'-end of said inserted fragment of DNA.

20. A method according to Claim 19 wherein said fragment of DNA is a cDNA molecule derived by reverse transcription from a viral RNA molecule and wherein said fragment is inserted in such a way that the end of the cDNA molecule that corresponds to the 5'-end of said viral RNA molecule is positioned adjacent to the 3'-end of said promoter.

21. A method according to Claim 20 wherein said fragment of DNA codes for rRNA, tRNA or snRNA.

22. An *in vitro* process for the preparation of a RNA transcript comprises an infectious viral RNA sequence transcript from a DNA fragment wherein the 5'-end of said RNA transcript is the same as the 5'-end of said DNA fragment, which comprises making a vector by a method according to Claim 10 and introducing said vector into an *in vitro* transcription system which contains an RNA polymerase which functions with said promoter so as to permit transcription of said DNA fragment thereby producing said RNA transcript.

23. A process according to Claim 22 wherein said RNA transcript comprises an infectious viral RNA sequence.

**Patentansprüche**

1. Rekombinanter DNA Vektor mit
(a) einem Replikationsursprung und einem selektierbaren Marker sowie
(b) einer Nucleotidsequenz, die einen Promotor enthält, in dem eine Transkriptionsinitiationsstelle modifiziert wurde zur Einführung einer nicht natürlich vorkommenden, glattendigen Restriktionsendonuclease-Erkennungssequenz, die so angeordnet ist, daß eine Spaltungsstelle dieser Restriktionsendonuclease-Erkennungssequenz mit der Transkriptionsinitiationsstelle des genannten Promotors zusammenfällt.

2. Rekombinanter DNA Vektor nach Anspruch 1, in welchem die genannte Restriktionsendonuclease-Erkennungssequenz eine *Sma*I Restriktionsendonuclease-Erkennungssequenz ist.

3. Rekombinanter DNA Vektor nach irgendeinem der Ansprüche 1 oder 2, in welchem der genannte Promotor ein Bakteriophagenpromotor ist.

4. Rekombinanter DNA Vektor nach Anspruch 3, in welchem der genannte Promotor der lambda $P_R$ Promotor ist.

5. Rekombinanter DNA Vektor nach irgendeinem der Ansprüche 1 oder 2, in welchem der genannte Promotor ein bakterieller Promotor ist.

6. Rekombinanter DNA Vektor nach irgendeinem der vorhergehenden Ansprüche, in welchem der genannte Replikationsursprung und der genannte selektierbare Marker jene von pUC9 oder M13mp9 sind.

7. Rekombinanter DNA Vektor nach irgendeinem der vorhergehenden Ansprüche, in welchem dieser Vektor weiters eine singuläre zweite Restriktionsendonuclease-Erkennungssequenz in einer Position unmittelbar 3' zu der genannten Restriktionsendonuclease-Erkennungssequenz enthält, deren Spaltungsstelle mit der Transkriptionsinitiationsstelle zusammenfällt.

8. Rekombinanter DNA Vektor nach Anspruch 7, in welchem diese singuläre zweite Restriktionsendonuclease-Erkennungssequenz eine *Eco*RI Restriktionsendonuclease-Erkennungssequenz ist.

9. Rekombinanter DNA Vektor pPM1 nach der Definition von Beispiel 4.

10. Bakterienstamm, der einen rekombinanten DNA Vektor nach irgendeinem der vorhergehenden Anpsrüche enthält und in sich repliziert.

11. Bakterienstamm nach Anspruch 10, in welchem der transformierte Stamm *Escherichia coli* K12 JM101 oder *Escherichia coli* K12 JM103 ist.

12. Verfahren zur Konstruktion eines rekombinanten DNA Vektors, der
(a) einen Replikationsursprung und einen selektierbaren Marker sowie
(b) eine einen Promotor enthaltende Nucleotidsequenz aufweist,
welches Verfahren die Stufe der Modifizierung der Transkriptionsinitiationsstelle dieses Promotors zur Einführung einer glattendigen Restriktionsendonuclease-Erkennungssequenz umfaßt, sodaß eine Spaltungsstelle dieser Restriktionsendonuclease-Erkennungssequenz mit der Transkriptionsinitiationsstelle dieses Promotors zusammenfällt.

13. Verfahren nach Anspruch 12, das die folgenden Stufen umfaßt:
(a) Isolierung eines DNA Fragments, das einen Promotor enthält, dessen Transkriptionsinitiationsstelle bekannt ist.
(b) Einsetzen dieses einen Promotor enthaltenden DNA Fragments in die replikative Form eines filamentösen einzelstrangigen Phagenstamms zur Herstellung eines rekombinanten filamentösen einzelstrangigen Phagen,

(c) Transformierung eines Bakterienstamms mit diesem rekombinanten Phagen,

(d) Isolierung der ssDNA dieses rekombinanten Phagen, die von dem Bakterienstamm ausgeschieden wird,

(e) Synthese eines unpassenden (mismatched) Oligonucleotid-Primers, der in der Nachbarschaft dieser Transkriptionsinitiationsstelle zu diesem Promotor teilweise komplementär ist und das Komplement einer gesamten oder eines Teils einer Restriktionsendonuclease-Erkennungssequenz enthält, wobei dieses Komplement der Restriktionsendonuclease-Erkennungssequenz oder ein Teil desselben innerhalb dieses Oligonucleotid-Primers angeordnet ist, sodaß die Spaltungsstelle dieses Komplements der Restriktionsendonucleasesequenz mit der Transkriptionsinitiationsstelle zusammenfällt,

(f) Replikation dieser ssDNA unter Verwendung des Oligonucleotid-Primers zur Herstellung eines modifizierten komplementären DNA Strangs, der einen modifizierten Promotor enthält, wobei dieser modifizierte Promotor jener ist, der die zu dem Oligonucleotid-Primer komplementäre Sequenz enthält,

(g) Replikation dieses modifizierten komplementären Strangs zur Herstellung eines doppelstrangigen DNA Fragments, das diesen modifizierten Promotor enthält,

(h) Einsetzung dieses den modifizierten Promotor enthaltenden DNA Fragments in ein DNA Vektorplasmid, das einen Replikationsursprung und einen selektierbaren Marker enthält, wodurch ein unvierseller Transkriptionsvektor konstruiert wird.

14. Verfahren nach Anspruch 13, bei welchem dieses einen Promotor enthaltende DNA Fragment ein cl-P M-P Fragment des Phagen lambda ist.

15. Verfahren nach Anspruch 13, bei welchem der filamentöse einzelstrangige Phagenstamm M13mp9 ist.

16. Verfahren nach irgendeinem der Ansprüche 13 bis 15, bei welchem dieser Bakterienstamm *Escherichia coli* K12 JM101 oder *Escherichia coli* K12 JM102 ist.

17. Verfahren nach Anspruch 13, bei welchem dieser Oligonucleotid-Primer 5'-OH-d(GGGACCATTAT-CACC)-3'-OH ist.

18. Verfahren nach Anspruch 13, bei welchem dieses DNA Vektorplasmid pUC9 ist.

19. Verfahren zur Herstellung eines rekombinanten DNA Vektors, bei welchem (a) ein Vektor nach irgendeinem der Ansprüche 1 bis 9 oder ein Vektor, der nach dem Verfahren nach irgendeinem der Ansprüche 12 bis 17 konstruiert ist, zur Verfügung gestellt wird, (b) dieser Vektor mit der Restriktionsendonuclease gespalten wird und (c) ein DNA Fragment an der Spaltungsstelle dieser Restriktionsendonuclease-Erkennungsstelle eingesetzt wird, sodaß das erste transkribierte Nucleotid das erste Nucleotid des 5'-Endes dieses eingesetzten DNA Fragments ist.

20. Verfahren nach Anspruch 19, bei welchem das DNA Fragment ein durch reverse Transkription von einem viralen RNA Molekül abgeleitetes cDNA Molekül ist und dieses Fragment so eingesetzt wird, daß das Ende des cDNA Moleküls, das dem 5'-Ende dieses viralen RNA Moleküls entspricht, anschließend and as 3'-Ende des Promotors angeordnet ist.

21. Verfahren nach Anspruch 20, bei welchem dieses DNA-Fragment für rRNA, t RNA oder snRNA codiert.

22. *In vitro* Verfahren zur Herstellung eines RNA Transkripts, das ein infektiöses, virales RNA Sequenz-Transkript von einem DNA Fragment enthält, wobei das 5'-Ende dieses RNA Transkripts das gleiche ist wie das 5'-Ende des DNA Fragments, bei welchem Verfahren ein Vektor nach einem Verfahren nach Anspruch 9, hergestellt und in ein *in vitro* Transkriptionssystem eingesetzt wird, welches eine RNA Polymerase enthält, die mit diesem Promotor so zusammenwirkt, daß Transkription des DNA Fragments ermöglicht und dabei das RNA Transkript produziert wird.

23. Verfahren nach Anspruch 22, wobei dieses RNA Transkript eine infektiöse virale RNA Sequenz enthält.

## Revendications

1. Vector d'ADN recombinant, comprenant
(a) une origine de réplication et un marqueur sélectionnable, et
(b) une séquence de nucléotides contenant un promoteur, dont un site d'initiation de la transcription a été modifié pour introduire une séquence non naturelle de reconnaissance de l'endonucléase de restriction à extrémité inactivée placée de telle manière qu'un site de clivage de ladite séquence de reconnaissance de l'endonucléase de restriction coïncide avec le site d'initiation de la transcription dudit promoteur.

2. Vecteur d'ADN recombinant selon la revendication 1, dans lequel ladite séquence de reconnaissance de l'endonucléase de restriction est une séquence de reconnaissance de l'endonucléase de restriction *Sma*l.

3. Vecteur d'ADN recombinant selon l'une quelconque des revendications 1 et 2, dans lequel ledit promoteur est un promoteur de bactériophage.

4. Vecteur d'ADN recombinant selon la revendication 3, dans lequel ledit promoteur est le promoteur lambda $P_R$.

5. Vecteur d'ADN recombinant selon l'une quelconque des revendications 1 et 2, dans lequel ledit promoteur est un promoteur bactérien.

6. Vecteur d'ADN recombinant selon l'une quelconque des revendications précédentes, dans lequel ladite origine de réplication et ledit marqueur sélectionnable sont ceux de pUC9 ou de M13mp9.

7. Vecteur d'ADN recombinant selon l'une quelconque des revendications précédentes, dans lequel ledit vecteur comprend en outre une seconde séquence de reconnaissance de l'endonucléase de restriction unique placée immédiatement en 3' par rapport à ladite séquence de reconnaissance de l'endonucléase de restriction dont le site de clivage coïncide avec ledit site d'initiation de la transcription.

8. Vecteur d'ADN recombinant selon la revendication 7, dans lequel ladite seconde séquence de reconnaissance de l'endonucléase de restriction unique est une séquence de reconnaissance de l'endonucléase de restriction *Eco*RI.

9. Vecteur d'ADN recombinant pPM1 défini dans l'exemple 4.

10. Souche bactérienne contenant et répliquant un vecteur d'ADN recombinant selon l'une quelconque des revendications précédentes.

11. Souche bactérienne selon la revendication 10, dans laquelle la souche qui est transformée est *Escherichia coli* K12 JM101 ou *Escherichia coli* K12 JM103.

12. Procédé de construction d'un vecteur d'ADN recombinant comprenant

(a) une origine de réplication et un marqueur sélectionnable, et

(b) une séquence de nucléotides contenant un promoteur, ce procédé incluant l'étape de modification du site d'initiation de la transcription dudit promoteur pour introduire une séquence de reconnaissance de l'endonucléase de restriction à extrémité inactivée de telle manière qu'un site de clivage de ladite séquence de reconnaissance de l'endonucléase de restriction coïncide avec le site d'initiation de la transcription dudit promoteur.

13. Procédé selon la revendication 12, comprenant les étapes qui consistent à:

(a) isoler un fragment d'ADN contenant un promoteur, dont le site d'initiation de la transcription est connu,

(b) insérer ledit fragment d'ADN contenant un promoteur dans la forme réplicative d'une souche de phage monocaténaire filamenteux pour produire un phage monocaténaire filamenteux recombinant,

(c) transformer une souche bactérienne avec ledit phage recombinant,

(d) isoler un ADNss dudit phage recombinant excrété par ladite souche bactérienne,

(e) synthétiser une amorce oligonucléotide dépareillée partiellement complémentaire dudit promoteur au voisinage dudit site d'initiation de la transcription et contenant le complément de la totalité ou d'une partie d'une séquence de reconnaissance de l'endonucléase de restriction, ledit complément de ladite séquence de reconnaissance de l'endonucléase de restriction ou de sa partie étant placé dans ladite amorce oligonucléotide de telle façon que le site de clivage dudit complément de ladite séquence de reconnaissance de l'endonucléase de restriction coïncide avec ledit site d'initiation de la transcription,

(f) répliquer ledit ADNss en utilisant ladite amorce oligonucléotide, pour produire un brin d'ADN complémentaire modifié contenant un promoteur modifié, ledit promoteur modifié étant le promoteur comprenant la séquence complémentaire de ladite amorce oligonucléotide,

(g) répliquer ledit brin complémentaire modifié pour produire un fragment d'ADN bicaténaire contenant ledit promoteur modifié,

(h) introduire ledit fragment d'ADN contenant ledit promoteur modifié dans un vecteur d'ADN plasmide qui contient une origine de réplication et un marqueur sélectionnable, pour construire ainsi un vecteur de transcription universel.

14. Procédé selon la revendication 13, dans lequel ledit fragment d'ADN contenant un promoteur est un fragment cl-$P_R$M-$P_R$ provenant du phage lambda.

15. Procédé selon la revendication 13, dans lequel ladite souche de phage monocaténaire filamentaux est M13mp9.

16. Procédé selon l'une quelconque des revendications 13 à 15, dans lequel ladite souche bactérienne est *Escherichia coli* K12 JM101 ou *Escherichia coli* K12 JM102.

17. Procédé selon la revendication 13, dans lequel ladite amorce oligonucléotide est 5'-OH-d(GGGACCATTATCACC)-3'-OH.

18. Procédé selon la revendication 13, dans lequel ledit vecteur d'ADN plasmide est pUC9.

19. Procédé de fabrication d'un vecteur d'ADN recombinant, comprenant les étapes qui consistent (a) à obtenir un vecteur selon l'une quelconque des revendications 1 à 9 ou un vecteur construit selon le procédé de l'une quelconque des revendications 12 à 17, (b) à cliver ledit vecteur avec ladite endonucléase de restriction, et (c) à insérer un fragment d'ADN au site de clivage dudit site de reconnaissance de l'endonucléase de restriction, pour que le premier nucléotide qui sera transcrit soit le premier nucléotide de l'extrémité 5' dudit fragment d'ADN inséré.

20. Procédé selon la revendication 19, dans lequel ledit fragment d'ADN est une molécule d'ADNc dérivée par transcription inverse d'une molécule d'ARN virale et dans lequel ledit fragment est inséré de manière à ce que l'extrémité de la molécule d'ADNc qui correspond à l'extrémité 5' de ladite molécule d'ARN viral soit placée en position adjacente à l'extrémité 3' dudit promoteur.

21. Procédé selon la revendication 20, dans lequel ledit fragment d'ADN porte le code d'un ARNr, d'un ARNt ou d'un ARNsn.

22. Procédé in vitro de préparation d'un transcript d'ARN comprenant un transcript de séquence d'ARN viral infectieux provenant d'un fragment d'ADN, dans lequel l'extrémité 5' dudit transcript d'ARN est la même que l'extrémité 5' dudit fragment d'ADN, qui comprend la fabrication d'un vecteur par un procédé selon la revendication 9 et l'introduction dudit vecteur dans un système de transcription in vitro qui contient une ARN polymérase dont le rôle est, avec ledit promoteur, de permettre la transcription dudit fragment d'ADN pour produire ledit transcript d'ARN.

23. Procédé selon la revendication 22, dans lequel ledit transcript d'ARN comprend une séquence d'ARN viral infectieux.

(FROM C. QUEEN (1983) J. MOL.)

APPL. GENET. 2:1-10

FIG. 1

Map of EcoRI — linearized pB3PR13

## FIG. 2

a.   Pr promoter/polylinker fusion in pPR1
     (a progenitor of pB3PR13)

Transcription
Start                                    Bam HI   Sma I   EcoRI

TTGACTATTTTACCTCTGGCGGTGATAATGGTTGCATGTACTAAGGAGGTTGTATGGATCCCGGGAATTC

21
See Fig. 1

b.   Pm promoter/polylinker fusion in pPM1

TTGACTATTTTACCTCTGGCGGTGATAATGGTCCCGGGAATTC

Sma I   EcoRI

# FIG. 3

FIG. 4

λ DNA

P_lac

P_RM  P_R

M I3/PRI
SS DNA

Pst I

Sma I

P_R

TGGCGGTGATAATGGTTGC ATGTACT
CCACTATTACCA GGG
(3') HO                (5')

T G
C    G C
T    C·G T
C·A·T G
A·T A T
T·T A A
T·T T G T
T·A A T
A·T G G
T·C·A·T
C·A
A·T

P_R

TCTTGTGTTAATGGT TTCTTT — (55b.) — GTGTTG  TGC ATGTACT
CACTATTACCA GGG
(3') HO C                  (5')

FIG. 5

EP 0 153 154 B1